(19) Europäisches Patentamt
European Patent Office
Office européen des brevets

(11) Veröffentlichungsnummer: 0 319 817
A2

(12) # EUROPÄISCHE PATENTANMELDUNG

(21) Anmeldenummer: 88119786.7

(22) Anmeldetag: 28.11.88

(51) Int. Cl.4: C07D 213/74 , C07D 213/38 , A61K 7/13

(30) Priorität: 05.12.87 DE 3741236

(43) Veröffentlichungstag der Anmeldung: 14.06.89 Patentblatt 89/24

(84) Benannte Vertragsstaaten: AT BE CH DE ES FR GB GR IT LI LU NL SE

(71) Anmelder: Henkel Kommanditgesellschaft auf Aktien
Postfach 1100 Henkelstrasse 67
D-4000 Düsseldorf 1(DE)

(72) Erfinder: Konrad, Günther, Dr.
Feuerbachweg 12
D-4010 Hilden(DE)
Erfinder: Lieske, Edgar
Hunsrückenstrasse 40
D-4000 Düsseldorf(DE)

(54) **Phenylamino- und Phenylaminomethyl-pyridine und diese Enthaltende Haarfärbemittel.**

(57)

(I) 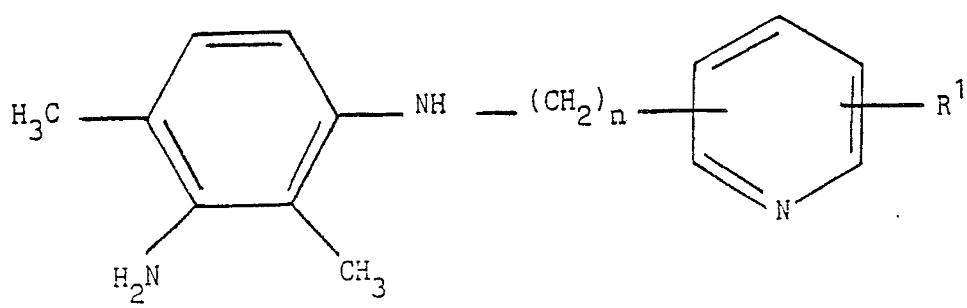

in der n = 0 oder 1 und $R^1$ = Wasserstoff, eine Alkyl- oder Hydroxyalkylgruppe mit 1 - 4 C-Atomen oder eine Aminogruppe ist, oder deren Salze eignen sich als Kuppler für Oxidationshaarfärbemittel auf Basis üblicher Entwicklerkomponenten. Sie erzeugen z. B. mit 2,4,5,6-Tetraaminopyrimidin rote bis braune und mit p-Toluylendiamin rote bis blauviolette Haaranfärbungen.

EP 0 319 817 A2

## Phenylamino- und Phenylaminomethyl-pyridine und diese enthaltende Haarfärbemittel

Gegenstand der Erfindung sind 3-Amino-2,4-dimethylphenylamino-pyridine und 3-Amino-2,4-dimethylphenylaminomethyl-pyridine (im folgenden 3-Amino-2,4-dimethylphenylamino-(methyl)-pyridine genannt) und deren Verwendung zur Herstellung von Haarfärbemitteln auf Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopryidinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen, ohne die Kopfhaut zu stark anzufärben. Auch soll der Farbaufzug gleichmäßig erfolgen, d.h. die stärker strapazierten Haarspitzen sollen nicht stärker gefärbt werden als der wenig geschädigte Haaransatz. Die damit erzeugten Färbungen müssen eine hohe Stabilität gegen Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Aminopyridin-Derivate sind als Oxidationsfarbstoffvorprodukte z. B. aus Fette, Seifen, Anstrichmittel 67. Jahrg. (1965), Seite 222 und 69. Jahrg. (1967), Seite 348 - 352 sowie aus US-PS-3.647.351 bekannt. 2,4-Diamino-m-xylole sind aus DE-A-31 37 473 ebenfalls bereits als Kuppler für Oxidationsfärbemittel bekannt. Die mit den bekannten Kupplern und Entwicklern bestellbaren Haarfärbemittel befriedigen jedoch nicht in bezug auf die Echtheitseigenschaften der damit erzielbaren Haaranfärbungen. Insbesondere sind die bekannten Aminopyridine und 2,4-Diamino-m-xylole nicht als Rotkuppler für Entwicklerkomponenten vom Typ der p-Phenylen-diamin-Derivate (insbesondere p-Phenylendiamin und p-Toluylendiamin) geeignet, da sie mit den Entwicklern dieser Substanzklasse entweder keine roten Nuancen ausbilden oder weil die gebildeten Farbnuancen nicht gleichmäßig auf Haaransatz und Haarspitzen aufziehen. Technisch brauchbare Rotkuppler für diese Entwickler sind bisher überhaupt nicht verfügbar.

Es wurden nun neue Aminopyridinderivate gefunden, die sich als Rotkuppler für p-Phenylendiamin, p-Toluylendiamin und Derivate dieser bekannten Entwicklerverbindungen eignen und überraschend gute anwendungstechnische Eigenschaften zeigen.

Gegenstand der Erfindung sind 3-Amino-2,4-dimethylphenylamino-(methyl)-pyridine der Formel I

(I) 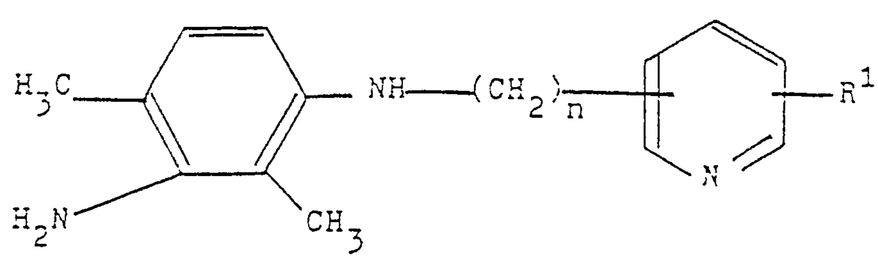

in der n = 0 oder 1 und $R^1$ Wasserstoff, eine Alkyl- oder Hydroxyalkylgruppe mit 1-4 C-Atomen oder eine Aminogruppe ist oder deren Salze.

Die 3-Amino-2,4-dimethylphenylamino-(methyl)-pyridine der Formel I sind als Kupplersubstanzen für eine Vielzahl bekannter Entwicklerverbindungen geeignet und erzeugen besonders brillante Färbungen mit hoher Licht- und Wärmestabilität. Als Entwicklersubstanzen können in den erfindungsgemäßen Haarfärbemitteln z. B. aromatische Amine mit einer oder mehreren weiteren $NH_2$-Gruppen, NHR-Gruppen, $NR_2$-Gruppen, wobei R eine Alkylgruppe mit 1-4 C-Atomen oder eine Hydroxyalkylgruppe oder eine Aminoalkylgruppe mit 2-4 C-Atomen darstellt, Aminophenole, Aminophenolether, Diaminopyridinderivate oder 2,4,5,6-Tetraaminopyrimidin und dessen Derivate eingesetzt werden.

Bevorzugt, insbesondere wegen ihrer leichten Zugänglichkeit, werden solche Verbindungen der Formel (I) eingesetzt, in welchem $R^1$ Wasserstoff oder eine Aminogruppe ist.

Die beiden Substituenten am Pyridinkern können beliebige Positionen zueinander einnehmen. Die Verbindungen der allgemeinen Formel (I) sind neu, sie lassen sich nach ansich bekannten Methoden der präparativen Chemie aus bekannten Ausgangsstoffen herstellen.

So werden 3-Amino-2,4-dimethylphenylamino-pyridine der Formel I, in welchen n = 0 ist, am einfachsten in der Weise gewonnen, daß man 2,4-Dimethyl-m-phenylen-diamin oder 2,4-Dimethyl-3-nitroanilin mit einer Verbindung der Formel (II)

(II) 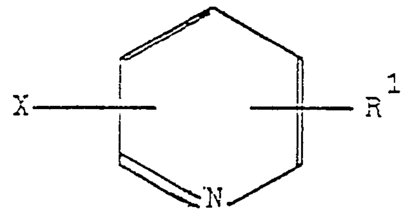

in der X = Chlor oder Brom und $R^1$ = Wasserstoff, eine Alkyl-oder Hydroxyalkylgruppe mit 1-4 C-Atomen oder eine Nitropruppe ist, in Gegenwart einer starken Base zur Umsetzung bringt und in dem Umsetzungsprodukt gegebenenfalls vorhandene Nitrogruppen katalytisch zur Aminogruppe reduziert.

3-Amino-2,4-dimethylphenylaminomethyl-pyridine der Formel I, in welchen n = 1 ist, lassen sich am besten dadurch gewinnen, daß man 2,4-Dimethyl-3-nitroanilin mit einem Pyridinaldehyd der Formel (III)

(III) 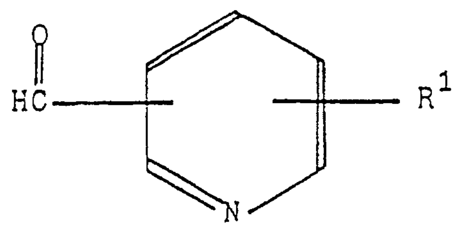

in der $R^1$ Wasserstoff, eine Alkyl- oder Hydroxyalkylgruppe mit 1-4 C-Atomen oder eine Nitropruppe ist, zur Schiffschen Base kondensiert und dann die Azomethingruppe und die Nitrogruppen gleichzeitig katalytisch hydriert.

Die Herstellung von erfindungsgemäßen Verbindungen der Formel I in welcher n = 0 ist, wird in den Beispielen 1.1 bis 1.3 und von Verbindungen der Formel I, in welchen n = 1 ist, in den Beispielen 1.4 bis 1.6 näher erläutert.

Ein weiterer Gegenstand der Erfindung sind Haarfärbemittel, enthaltend Oxidationsfarbstoffvorprodukte in einem kosmetischen Träger, wobei als Oxidationsfarbstoffvorprodukte 3-Amino-2,4-dimethylphenylamino-(methyl)-pyridine der allgemeinen Formel I als Kuppler sowie bekannte Entwickler- und Kupplersubstanzen enthalten sind.

Für die erfidungsgemäßen Haarfärbemittel eigenen sich von den erfindungsgemäßen Verbindungen der Formel I besonders bevorzugt solche, in welchen n = 0 ist. Von den bekannten Entwicklern sind besonders bevorzugt solche geeignet, die mit den erfindungsgemäßen Kupplern rote, rotbraune oder rotviolette Nuancen ausbilden; dies sind in erster Linie p-Phenylendiamin, p-Toluylen-diamin und deren Derivate.

In die erfindungsgemäßen Haarfärbemitte können die 3-Amino-2,4-dimethylphenylamino-(methyl)-pyridine entweder in freier Form oder auch in Form von Salzen der Aminogruppen mit anorganischen oder organischen Säuren, z. B. in Form der Hydrochloride, Sulfate, Phosphate, Acetate, Propionate, Lactate oder Citrate, eingesetzt werden.

Die erfindungsgemäßen Haarfärbemittel können neben den 3-Amino-2,4-dimethylphenylamino-(methyl)-pyridinen der Formel (I) auch andere bekannte Kupplersubstanzen enthalten, die zur Modifizierung der Farbnuancen und zur Erzeugung natürlicher Farbtöne erforderlich sind. Solche üblichen Kupplerverbindungen sind z. B. andere m-Phenylendiamine, z. B. 2,4-Diaminophenyl-2-hydroxyethylether, 1,3-Bis-2,4-diaminophenoxypropan, 2,4-Diaminoanisol oder Phenole, Resorcine, m-Aminophenole, Naphthole oder Pyrazolone.

Gegebenenfalls können auch direktziehende Farbstoffe zusätzlich zur weiteren Modifizierung der Farbnuancen eingesetzt werden. Solche direktziehenden Farbstoffe sind z. B. Nitrophenylendiamine, Nitroaminophenole, Anthrachinonfarbstoffe oder Indophenole.

Zu den erfindungsgemäßen Haarfärbemitteln werden die neuen Kuppler der Formel (I) und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die neuen Kuppler der Formel (I) sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel wie anionische, nichtionische oder ampholytische Tenside, z. B. Seifen, Fettalkoholsulfate, Alkansulfonate, alpha-Olefinsulfonate, Fettalkoholpolyglykolethersulfate, Ethylenoxidanlagerungsprodukte an Fettalkohole, an Fettsäuren und an Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettkomponenten wie z. B. Fettalkohole, Paraffinöle oder Fettsäureester ferner Parfümöle und haarpflegende Zusätze, wie z. B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z. B. werden Emulgiermittel in Konzentrationen von 0,5 - 30 Gew.-% und Verdickungsmittel in Konzentrationen von 0,1 - 25 Gew.-% des gesamten Färbemittels eingesetzt.

Besonders geeignet als Träger ist eine Öl-in-Wasser-Emulsion mit einem Gehalt von 0,1 - 25 % Gew.-% einer Fettkomponente und 0,5 - 30 Gew.-% eines Emulgiermittels aus der Gruppe der anionischen, nichtionischen oder ampholytischen Tenside.

Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an 3-Amino-2,4-dimethylphenylamino-(methyl)-pyridinen gemäß Formel I kann in den erfindungsgemäßen Haarfärbemitteln etwa 0,05 - 10 Millimol pro 100 g des Haarfärbemittels betragen.

Eine bevorzugte Anwendungsform für die erfindungsgemäßen 3-Amino-2,4-dimethylphenylamino-(methyl)-pyridine der Formel (I) sind Cremehaarfärbemittel in Form einer Öl-in-Wasser-Emulsion mit einem Gehalt von

1 bis 10 Millimol pro 100 g an Entwicklerkomponenten,
1 bis 10 Millimpl pro 100 g an Kupplerkomponenten
1 bis 10 Gew.-% eines Alkyl($C_{10}$-$C_{18}$)-sulfat- oder Alkyl($C_{10}$-$C_{16}$)-ethersulfattensids,
5 bis 20 Gew.-% eines Fettalkohols oder Fettalkoholgemisches mit 12 bis 18 C-Atomen,
0.1 bis 2 Gew.-% eines Oxidationsinhibitors, bevorzugt aus der Gruppe Alkalisulfit, Alkaliascorbat oder Alkali-dithionit
sowie Ammoniak in einer Menge, um den pH-Wert der Emulsion auf einen Wert zwischen 8 und 10 einzustellen.

Das genannte Alkylsulfat- oder Alkylethersulfattensid kann als Alkali-, Ammonium- oder Alkanolammoniumsalz mit 2 oder 3 C-Ato men in der Alkanolgruppe vorliegen, z. B. als Natrium-, Triethanolamin oder Isopropanolammoniumsalz. Als Alkyl-$C_{10}$-$C_{16}$-ethersulfattensid kann ein Schwefelsäuremonoestersalz eines Anlagerungsproduktes von 1 bis 10 Mol Ethylenoxid an einen $C_{10}$-$C_{16}$-Fettalkohol eingesetzt werden.

Die oxidative Entwicklung der Färbung kann grundsätzlich mit Luftsauerstoff erfolgen. Bevorzugt wird

jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Die Anwendung der erfindungsgemäßen Haarfärbemittel kann, unabhängig von der Art der kosmetischen Zubereitung, z. B. als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen.

Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo verwendet wurde.

Die nachfolgenden Beispiele sollen den Erfindungsgegenstand näher erläutern, ohne ihn jedoch hierauf zu beschränken.

## Beispiele

1. Herstellungsbeispiele

### 1.1 2- (3-Amino-2,4-dimethylphenyl)-amino -pyridin

Stufe 1: 2- (3-Nitro-2,4-dimethylphenyl)-amino -pyridin

3,3 g 3-Nitro-2,4-dimethylanilin und 3,2 g 2-Brom-pyridin werden gemischt und die Mischung 8 Stunden auf 170 °C erhitzt. Nach dem Abkühlen auf 20 °C wurde die dabei gebildete schmierige Masse in Wasser gegeben und die Lösung durch Zugabe von Natriumcarbonat alkalisch eingestellt. Die dabei sich abscheidende Substanz wurde aus Ethanol umkristallisiert. Es wurden 2 g des Produktes mit einem Schmelzpunkt von 178 - 179 °C erhalten. Aus der wässrigen Phase wurden durch Einengen weitere 2,1 g der Substanz gewonnen.

Stufe 2:

3,7 g des Produktes aus Stufe 1 wurden in 150 ml einer Ethanol-Wasser-Mischung (1:1 Vol.) gelöst und in Gegenwart von Palladium (auf Aktivkohle) als Katalysator hydriert. Nach Aufnahme von 1,07 l Wasserstoff wurde die Lösung eingeengt und der Rückstand in Diethylether aufgenommen. Nach Abdampfen des Ethers wurden 2,4 g des Produktes mit einem Schmelzpunkt von 132 - 135 °C erhalten.

| Analyse: $C_{13}H_{15}N_3$ (MG: 213,3) | | | |
|---|---|---|---|
| | C | H | N |
| ber. | 73,21 | 7,09 | 19,70 |
| gef. | 73,10 | 7,19 | 19,30 |

### 1.2 2- (3-Amino-2,4-dimethylphenyl)-amino -3-aminopyridin

Stufe 1: 2- (3-Amino-2,4-dimethylphenyl)-amino -3-aminopyridin

8,4 g 2,4-Diamino-m-xylol, 9,6 g 2-Chlor-3-nitro-pyridin und 3,0 g Calciumcarbonat wurden in 300 ml

Wasser gegeben und die Mischung 8 Stunden unter Rückfluß zum Sieden erhitzt. Das Reaktionsgemisch wurde heiß filtriert, aus dem Filtrat schied sich beim Erkalten auf 20 °C das Reaktionsprodukt ab. Es wurde isoliert und aus Toluol umkristallisiert. Es wurden 6,2 g des Produktes vom Schmelzpunkt 173 -175 °C erhalten.

| Analyse $C_{13}H_{14}N_4O_2$ (MG: 258,3) | | | | |
|---|---|---|---|---|
| | C | H | N | O |
| ber. | 60,45 | 5,46 | 21,69 | 12,4 |
| gef. | 60,40 | 5,32 | 22,1 | 12,0 |

Stufe 2:

6,2 des Produktes aus Stufe 1 wurden in 150 ml Ethanol gelöst und in Gegenwart von Palladium (auf Aktivkohle) als Katalysator hydriert. Nach Aufnahme von 1,74 l Wasserstoff wurde die Lösung eingeengt und 3.8 g des Produktes mit einem Schmelzpunkt von 202 °C als Rückstand gewonnen.

| Analyse: $C_{13}H_{16}N_4$ (MG: 228,3) | | | |
|---|---|---|---|
| | C | H | N |
| ber. | 68,39 | 7,07 | 24,54 |
| gef. | 68,40 | 7,06 | 24,60 |

1.3 2- (3-Amino-2,4-dimethylphenyl)-amino -5-amino-pyridin-trihydrochlorid(-monohydrat)

Stufe 1: 2- (3-Amino-2,4-dimethyl)-amino -5-nitro-pyridin

4,2 g 2,4-Diamino-m-xylol und 4,8 g 2-Chlor-5-nitropyridin wurden zusammen mit 3,5 g Kaliumfluorid in 200 ml Wasser 8 Stunden lang am Rückfluß erhitzt. Das nach dem Erkalten ausgefallene Produkt wurde abgesaugt, mit Waser gewaschen, mit warmem Toluol aufgeschlämmt. Nach erneutem Absaugen resultierten nach Trocknen 4,5 g Substanz vom Schmelzpunkt 200 °C. Nach Umkristallisation aus Toluol wurden 3,2 g Produkt erhalten, das bei 208 - 9 °C schmolz

| Analyse: $C_{13}H_{14}N_4O_2$ (MG: 258,3) | | | |
|---|---|---|---|
| | C | H | N |
| ber. | 60,45 | 5,46 | 21,69 |
| gef. | 61,1 | 5,78 | 22,0 |

Stufe 2:

2.4 g des Produkts aus Stufe 1 wurden in 100 ml Ethanol mit Wasserstoff (Aufnahme 0,655 l) in Gegenwart von Raney-Nickel als Katalysator reduziert. Nach dem Abfiltrieren wurde rasch mit Salzsäure angesäuert und dann zur Trockene eingedampft; es wurden 1,2 g isoliert.

| Analyse: $C_{13}H_{15}N_4$ + 3HCl + $H_2O$ (MG: 354,7) | | | | |
|---|---|---|---|---|
| | C | H | N | Cl |
| ber: | 43,9 | 5,95 | 15,75 | 29,9 |
| gef: | 43,7 | 5,56 | 15,1 | 29,7 |

1.4 2- (3-Amino-2,4-dimethylphenyl)-amino -methyl)-pyridin-trihydrochlorid

Stufe 1: 2- (2,4-Dimethyl-3-nitrophenylimino)-methyl -pyridin

3,3 g 4-Amino-2-nitro-m-xylol und 2,1 g 2-Pyridinaldehyd wurden in 50 ml Ethanol stehengelassen. Die ausgeschiedenen Kristalle wurden abgesaugt und mit Ethanol gewaschen. Der Schmelzpunkt des getrockneten Produkts (3,0 g) betrug 114 - 116° C.

| Analyse: $C_{14}H_{13}N_3O_2$ (MG: 255,3) | | | |
|---|---|---|---|
| | C | H | N |
| ber. | 65,87 | 5,13 | 16,46 |
| gef. | 65,7 | 5,04 | 16,3 |

Stufe 2:

3,0 g des Produkts aus Stufe 1 wurden in 150 ml Ethanol und mit Platin (auf Aktivkohle) als Katalysator hydriert (1 l $H_2$). Nach Reduktion wurde mit Salzsäure angesäuert und zur Trockene eingedampft. Erhalten wurden 3,2 g sehr hygroskopisches Produkt mit einem Zersetzungspunkt von 122° C.

| Analyse: $C_{14}H_{17}N_3$ + 3HCl (MG: 336,7) | | | |
|---|---|---|---|
| | C | H | N |
| ber. | 49,94 | 5,99 | 12,48 |
| gef. | 49,0 | 6,07 | 12,1 |

1.5 3- (3-Amino-2,4-dimethylphenyl)-amino -methyl -pyridin-trihydrochlorid

Stufe 1: 3- (2,4-Dimethyl-3-nitrophenylimino) -methyl-pyridin

Analog der Stufe 1 von 1,4 wurden aus 3,3 g 4-Amino-2-nitro-m-xylol und 2,1 g 3-Pyridinaldehyd 3,6 g Produkt vom Schmelzpunkt 138 - 142° C erhalten.

| Analyse: $C_{14}H_{13}N_3O_2$ I. | | | |
|---|---|---|---|
| | C | H | N |
| ber. | 65,87 | 5,13 | 16,46 |
| gef. | 66,4 | 5,08 | 16,4 |

Stufe 2:

Reduktion von 3,6 g des Produkts aus Stufe 1 in 150 ml Ethanol mit Wasserstoff und Palladium (auf Aktivkohle) als Katalysator (1,32 l $H_2$) lieferten, nach Ansäuern mit Salzsäure und Eindampfen zur Trockene, 3,6 g einer ab 175° C schmelzenden sehr hygroskopischen Substanz.

| Analyse: $C_{14}H_{17}N_3$ + 3HCl + 1$H_2O$ (MG: 354,7) | | | |
|---|---|---|---|
| | C | H | N |
| ber. | 47,41 | 6,25 | 11,85 |
| gef. | 46,9 | 5,82 | 11,8 |

1.6 4- (3-Amino-2,4-dimethylphenyl)-amino -methyl -pyridin-trihydrochlorid (-dihydrat)

Stufe 1: 4- (2,4-Dimethyl-3-nitrophenylimino)-methyl pyridin

Analog der Stufe 1 von 1.4 wurden aus 3,3 g 4-Amino-2-nitro-m-xylol und 2,1 g 4-Pyridinaldehyd 2,3 g Produkt vom Schmelzpunkt 111 - 113° C erhalten.

| Analyse: $C_{14}H_{13}N_3O_2$ | | |
|---|---|---|
| | C | H |
| ber. | 65,87 | 5,13 |
| gef. | 66,3 | 5,29 |

Stufe 2:

Reduktion der 2,3 g des Produkts aus Stufe 1 in 150 ml Ethanol mit Wasserstoff und Palladium (auf Aktivkohle) als Katalysator (0,85 l $H_2$) lieferten, nach Ansäuern mit Salzsäure und Eindampfen zur Trockene, 2,3 g hygroskopisches Produkt.

| Analyse: $C_{14}H_{17}N_3$ + 3HCl + 2$H_2O$ | | | |
|---|---|---|---|
| | C | H | N |
| ber. | 45,12 | 6,49 | 11,27 |
| gef. | 45,10 | 6,08 | 11,10 |

2. Anwendungsbeispiel

Es wurden erfindungsgemäße Haarfärbemittel in Form einer Haarfärbe-Cremeemulsion der folgenden Zusammensetzung hergestellt:

| | |
|---|---|
| Fettalkohol $C_{12}$-$C_{18}$ | 10 g |
| Fettalkohol $C_{12}$-$C_{14}$ + 2EO-sulfat Na-Salz, 28%ig | 25 g |
| Wasser | 60 g |
| Entwicklerkomponente (Komponente E) | 7,5 mMol |
| Kupplerkomponente (Komponente K) | 7,5 mMol |
| $Na_2SO_3$ (Inhibitor) | 1,0 g |
| konzentrierte Amoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Amoniak-Lösung der pH-Wert der Emulsion auf 9,5 eingestellt, dann wurde mit Wasser auf 100 g aufgefüllt.

Die oxidative Entwicklung der Färbung wurde mit 3%iger Wassertoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27° C belassen. Nach Beendigung des Färbeprozesses wurde das Haar gespült, mit einem üblichen Haarwaschmittel ausgewaschen und anschließend getrocknet.

Als Entwicklerkomponenten wurden die folgenden Verbindungen eingesetzt:

E 1: 2,4,5,6-Tetraaminopyrimidin-(sulfat)
E 2: p-Toluylendiamin(-sulfat)
E 3: p-Phenylendiamin
E 4: p-Aminophenol
E 5: N,N-Dimethyl-p-phenylendiamin-sulfat
E 6: $N^1$- (2-Methansulfonamido)-ethyl -3-methyl-p-phenylendiamin-sesquisulfat
E 7: $N^1$-Ethyl-$N^-$-(2-hydroxyethyl)-p-phenylendiamin-sulfat
E 8: 2-Chlor-p-phenylendiamin
E 9: 2,5-Diaminoanisol-sulfat

Als Kupplerkomponente wurden die erfindungsgemäßen 3-Amino-2,4-dimethyl-phenylamino-(methyl)-pyridine gemäß Beispiel 1.1 bis 1.6 eingesetzt (K 1.1 bis K 1.6).

Die mit diesen Oxidationsfärbemittelvorprodukten in den Kombinationen gemäß Tabelle I erhaltenen Haaranfärbungen sind der Tabelle zu entnehmen.

Tabelle I

| Anwendungs-Beispiel | Entwicker-Komponente | Kuppler-Komponente | erhaltene Farbnuance |
|---|---|---|---|
| 2.1 | E 1 | K 1.1 | rotblond |
| 2.2 | E 1 | K 1.2 | rothaarig |
| 2.3 | E 1 | K 1.3 | olivbraun |
| 2.4 | E 1 | K 1.4 | rothaarig |
| 2.5 | E 1 | K 1.5 | kamelbraun |
| 2.6 | E 1 | K 1.6 | goldbraun |
| 2.7 | E 2 | K 1.1 | voilett |
| 2.8 | E 2 | K 1.2 | dunkelrotbraun |
| 2.9 | E 2 | K 1.3 | dunkelpurpur |
| 2.10 | E 2 | K 1.4 | rotbraun |
| 2.11 | E 2 | K 1.5 | purpur |
| 2.12 | E 2 | K 1.6 | blauviolett |
| 2.13 | E 3 | K 1.3 | dunkelpurpur |
| 2.14 | E 4 | K 1.3 | dunkelvoilett |
| 2.15 | E 5 | K 1.3 | violettbraun |
| 2.16 | E 6 | K 1.3 | dunkelvoilett |
| 2.17 | E 7 | K 1.3 | violett |
| 2.18 | E 8 | K 1.3 | rubin |
| 2.19 | E 9 | K 1.3 | dunkelblau |

## Ansprüche

1. 3-Amino-2,4-dimethylphenylamino-(methyl)-pyridine der allgemeinen Formel I

(I)

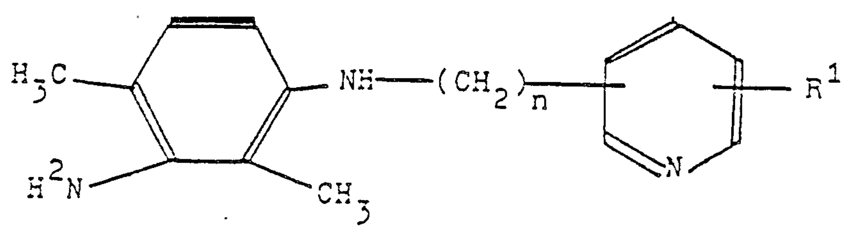

in der n = 0 oder 1 und $R^1$ Wasserstoff, eine Alkyl- oder Hydroxyalkylgruppe mit 1-4 C-Atomen oder eine Aminogruppe ist, oder deren Salze.

2. 3-Amino-2,4-dimethylphenylamino-(methyl)-pyridine nach Patentanspruch 1, dadurch gekennzeichnet, daß R' Wasserstoff oder eine Aminogruppe ist, oder deren Salze.

3. 3-Amino-2,4-dimethylphenylamino-(methyl)-pyridine nach Patentanspruch 1 oder 2, dadurch gekennzeichnet, daß n = 0 ist.

4. Verfahren zur Herstellung von 3-Amino-2,4-dimethylphenylamino-pyridinen der Formel I, gemäß Anspruch 1, worin n = 0 ist, dadurch gekennzeichnet, daß man 2,4-Dimethyl-m-phenylendiamin oder 2,4-Dimethyl-3-nitroamilin mit einer Verbindung der Formel II

(II)

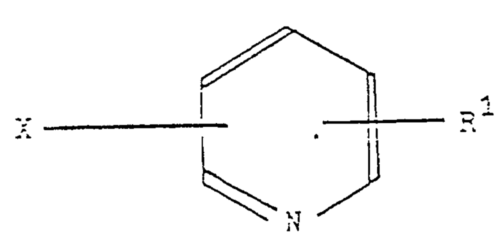

in der X = Chlor oder Brom und $R^1$ Wasserstoff, eine Alkyl-oder Hydroxyalkylgruppe mit 1-4 C-Atomen oder eine Nitrogruppe ist in Gegenwart einer starken Base zur Umsetzung bringt und in dem Umsetzungsprodukt gegebenenfalls vorhandenen Nitropruppen katalytisch reduziert.

5. Verfahren zur Herstellung von 3-Amino-2,4-dimethyl-phenylamino-methylpyridinen der Formel I gemäß Anspruch 1, worin n = 1 ist, dadurch gekennzeichnet, daß man 2,4-Dimethyl-3-nitroamilin mit einem Pyridinaldehyd der Formel III

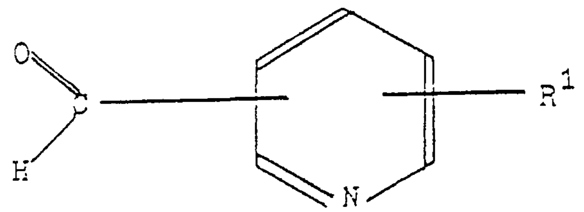

(III)

in der $R^1$ Wasserstoff eine Alkyl- oder Hydroxyalkylgruppe mit 1-4 C-Atomen oder eine Nitrogruppe ist, zur Schiffschen Base kondensiert und dann die Azomethingruppe und die Nitrogruppen gleichzeitig katalytisch hydriert.

6. Verwendung von 3-Amino-2,4-dimethylphenyl-amino(methyl)-pyridinen der Formel I nach Anspruch 1 in Oxidationsfärbemitteln als Kupplersubstanzen zusammen mit üblichen Entwicklersubstanzen.

7. Haarfärbemittel, enthaltend Oxidationsfarbstoffvorprodukte in einem kosmetischen Träger, dadurch gekennzeichnet, daß als Oxidationsfarbstoffvorprodukte 3-Amino-2,4-dimethylphenylamino-(methyl)-pyridine der allgemeinen Formel I nach Anspruch 1 in einer Menge von 0,05 bis 10 Millimol pro 100 g des Haarfärbemittels sowie bekannte Entwickler- und Kupplersubstanzen enthalten sind und daß Entwicklersubstanzen und Kupplersubstanzen im Molverhältnis 1 : 0,5 bis 1 : 2 enthalten sind.

8. Haarfärbemittel nach Anspruch 6, dadurch gekennzeichnet, daß als Träger eine Öl-in-Wasser-Emulsion mit einem Gehalt von 0,1 bis 25 Gew.-% einer Faltkomponente und 0,5 bis 30 Gew.-% eines Emulgators aus der Gruppe der anionischen, nichtionischen oder anpholytischen Tenside enthalten sind.